# EUROPÄISCHE PATENTSCHRIFT

(11) **EP 1 896 432 B1**
(45) Veröffentlichungstag und Bekanntmachung des Hinweises auf die Patenterteilung: **02.11.2011**
(21) Anmeldenummer: 06763482.4
(22) Anmeldetag: 02.06.2006
(51) Int. Cl.: C07D 251/70

(54) **VERFAHREN ZUR AUFBEREITUNG EINES ALKOXYCARBONYLAMINOTRIAZIN ENTHALTENDEN REAKTIONSGEMISCHES**
PROCESS FOR TREATING A REACTION MIXTURE COMPRISING ALKOXYCARBONYLAMINOTRIAZINE
PROCEDE DE PRODUCTION D'UN MELANGE REACTIONNEL RENFERMANT UNE ALCOXYCARBONYLAMINOTRIAZINE

(30) Priorität: 06.06.2005 DE 102005025899
(43) Veröffentlichungstag der Anmeldung: 12.03.2008
(73) Patentinhaber: BASF SE, 67056 Ludwigshafen (DE)
(72) Erfinder: SCHNEIDER, Jörg, B-1970 Wezembeek-Oppern (BE); SCHERR, Günter, 67065 Ludwigshafen (DE); EHRHARDT, Rainer, 68199 Mannheim (DE); EICHFELDER, Andreas, 67133 Maxdorf (DE); REIF, Martin, 67354 Römerberg (DE); HIRSCH, Stefan, 67435 Neustadt (DE); SIEDER, Georg, 67098 Bad Dürkheim (DE); HOLTMANN, Thomas, 67346 Speyer (DE); CIPRIAN, Jürgen, 67071 Ludwigshafen (DE); ASCHERL, Hermann, 67246 Dirmstein (DE)
(86) Internationale Anmeldenummer: PCT/EP2006/062861
(87) Internationale Veröffentlichungsnummer: WO 2006/131498

(56) Entgegenhaltungen:
- EP-A- 0 624 577
- WO-A-2003/035628
- WO-A-2004/054990

## Beschreibung

Die Erfindung betrifft ein Verfahren zur Aufarbeitung eines alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisches.

Die Herstellung von Alkoxycarbonylaminotriazinen durch Umsetzung von Triazinen, beispielsweise Melamin, mit Kohlensäureestern in Gegenwart einer Base ist zum Beispiel aus EP-A 0 624 577 bekannt. Hierbei wird in der Regel Melamin mit einem Kohlensäureester in Gegenwart des dem Kohlensäureester zugrunde liegenden Alkanols und in Gegenwart eines Alkalialkanolats, basierend auf dem dem Kohlensäureester zugrunde liegenden Alkohol, als Base zur Reaktion gebracht. Zur Aufbereitung wird dem Reaktionsgemisch eine mineralische Säure zur Neutralisation zugeführt. Als geeignete Säuren sind Phosphorsäure, Schwefelsäure und/oder Salzsäure genannt. Die Gewinnung des Alkoxycarbonylaminotriazins erfolgt anschließend durch eine Extraktion mit einem organischen Lösemittel und der Verdampfung des Lösemittels. Alternativ wird nach der Zugabe der Säure ein Feststoff durch Filtration isoliert, der dann gewaschen und getrocknet wird.

Aus der WO-A 03/035628 ist ein Verfahren zur Herstellung von Alkoxycarbonylaminotriazinen bekannt, bei welchem die Reaktionsmischung zur Aufbereitung zunächst mit einer bevorzugt wässrigen Säure neutralisiert wird. Als geeignete Säuren sind Salpetersäure, Schwefelsäure, Phosphorsäure oder deren Mischungen, aber auch Ameisensäure genannt. Nach der Zugabe der Säure zum Reaktionsgemisch bilden sich eine wässrige und eine alkanolische Phase, die voneinander getrennt werden. Die alkanolische Phase enthält dabei das Alkoxycarbonylaminotriazin. Zur Erhöhung der Konzentration an Alkoxycarbonylaminotriazin wird die organische Phase nach der Abtrennung der wässrigen Phase eingeengt.

Ein entsprechendes Verfahren zur Aufbereitung eines Alkoxycarbonylaminotriazin enthaltenden Reaktionsgemisches ist auch in WO-A 2004/054990 offenbart.

Aus WO-A 2004/041922 ist ein Herstellungs- und Aufbereitungsverfahren für Carbamat-Melamin-Formaldehyd-Vernetzer bekannt. Die Aufbereitung erfolgt hierbei ebenfalls durch Zugabe einer Säure, z. B. Schwefelsäure, Ameisensäure, Oxalsäure, Phosphorsäure, Salzsäure oder Mischungen daraus. Das bei der Neutralisation entstehende Salz wird durch Filtration und Waschen mit Wasser entfernt.

Aufgabe der vorliegenden Erfindung ist es, ein Verfahren zur Aufbereitung eines alkanolischen, mindestens ein Alkoxycarbonylaminotriazin enthaltenden Reaktionsgemisches bereitzustellen, welches eine Entfernung von Salzen und gegebenenfalls weiterer polarer Komponenten aus einem mindestens ein Alkoxycarbonylaminotriazin enthaltenden Reaktionsgemisch sowie die gezielte Beeinflussung mehrerer unterschiedlich polarer Komponenten im Reaktionsgemisch ohne großen apparativen Aufwand erlaubt.

Gelöst wird die Aufgabe durch ein Verfahren zur Aufbereitung eines alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisches, bei welchem polare und/oder ionische Komponenten durch Extraktion mit einem polaren, nicht vollständig mit der im Reaktionsgemisch vorhandenen organischen Phase mischbaren Extraktionsmittel entfernt werden, wodurch eine alkanolische, mindestens ein Alkoxycarbonylaminotriazin enthaltende Phase und eine polare, Extraktionsmittel mit darin gelösten polaren und/oder ionischen Komponenten enthaltende Phase erhalten werden, wobei die Extraktion in einer Mixer/Settler-Einheit, einer Extraktionskolonne, einem auf Basis der Zentrifugalfeldtrennung basierenden Extraktor oder einer Kombination daraus durchgeführt wird.

Bevorzugte Alkoxycarbonylaminotriazine sind solche der allgemeinen Formel (I) 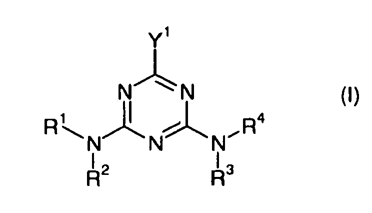 in der die Symbole und Indices die nachfolgende Bedeutung haben:
Y¹ Wasserstoff, C₁-C₄-Alkyl, gegebenenfalls durch C₁-C₄-Alkyl, C₁-C₄-Alkoxy oder Halogen substituiertes Phenyl oder ein Rest der Formel NR⁵R⁶ und
R¹, R², R³, R⁴, R⁵ und R⁶ unabhängig voneinander jeweils Wasserstoff oder ein Rest der Formel COOX oder X oder ausgewählt aus der Gruppe (-CH₂-O)₁,-H, (-CH₂-O)₁-R, (-CH₂-O)ₖ₋CH₂-N(Z)-Q und (-CH₂-O)ₖ-CH₂-N(Z)-Q, wobei
   - k für 0 bis 10, vorzugsweise 1 bis 5, mehr bevorzugt 1 oder 2 und insbesondere für 1 und I für 1 bis 10, vorzugsweise 1 bis 5, mehr bevorzugt für 1 oder 2 und insbesondere 1 steht,
   - R ausgewählt ist aus der Gruppe Alkyl, Cycloalkyl und Alkylaryl, wobei die Gruppen R vorzugsweise weniger als 13 Kohlenstoffatome enthalten und R bevorzugt ein C₁-C₁₃-Alkyl und besonders bevorzugt Methyl oder Butyl ist,
   - Q ein Triazin-Rest der allgemeinen Formel (II) 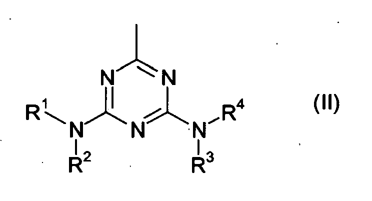 ist,
   - X für C₁-C₁₃-Alkyl, dessen Kohlenstoffgerüst durch 1 oder 2 nicht benachbarte Sauerstoffatome in Etherfunktion unterbrochen und/oder durch Hydroxy substituiert sein kann oder für C₃-C₆-Alkenyl steht und
   - Z für einen Rest R¹, R², R³, R⁴, R⁵ oder R⁶, wie oben definiert, steht
und
mindestens einer der Reste R¹ bis R⁴, oder wenn Y¹ für NR⁵R⁶ steht, mindestens einer der Reste R¹ bis R⁶ COOX.

Dabei bedeutet C₁-C₄-Alkyl z. B. Methyl, Ethyl, Propyl, Isopropyl, Butyl, sec-Butyl oder tert-Butyl.

Gegebenenfalls durch C₁-C₄-Alkyl, C₁-C₄-Alkoxy oder Halogen substituiertes Phenyl sind z. B. Phenyl, 2-, 3- oder 4-Methylphenyl, 2-, 3- oder 4-Ethylphenyl, 2,4-Dimethylphenyl, 2-, 3- oder 4-Methoxyphenyl, 2-, 3- oder 4-Ethoxyphenyl, 2,4-Dimethoxyphenyl, 2-, 3- oder 4-Fluorphenyl oder 2-, 3- oder 4-Chlorphenyl.

C₁-C₁₃-Alkyl, dessen Kohlenstoffgerüst durch 1 oder 2 nicht benachbarte Sauerstoffatome in Etherfunktion unterbrochen und/oder durch Hydroxy substituiert sein kann, bedeutet z. B. Pentyl, Isopentyl, Neopentyl, tert-Pentyl, Hexyl, 2-Methylpentyl, Heptyl, Octyl, 2-Ehtylhexyl, Isooctyl, Nonyl, Isononyl, Decyl, Isodecyl, Undecyl, Dodecyl, Tridecyl, Isotridecyl, 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 4-Methoxybutyl, 2- oder 4-Ethoxybutyl, 3,6-Dioxaheptyl, 3,6-Dioxaoctyl, 3,7-Dioxaoctyl, 4,7-Dioxaoctyl, 2- oder 3-Butoxypropyl, 2- oder 4-Butoxybutyl, 2-Hydroxyethyl, 2- oder 3-Hydroxypropyl, 2- oder 4-Hydroxybutyl, 3-Hydroxybut-2-yl. (Die obigen Bezeichnungen Isooctyl, Isononyl, Isodecyl und Isotridecyl sind Trivialbezeichnungen und stammen von den nach der Oxosynthese erhaltenen Alkoholen - vgl. Ullmanns Encyclopedia of Industrial Chemistry, 5th Edition, Vol. A1, Seiten 290 bis 293, sowie Vol. A10, Seiten 284 und 285.)

C₃-C₆-Alkenyl bedeutet z. B. Allyl, Methallyl, Ethallyl, 2-, 3- oder 4-Penten-1-yl oder 2-, 3-, 4- oder 5-Hexen-1-yl.

Gegebenenfalls ein oder zwei nicht benachbarte Sauerstoffatome als Etherbindung enthaltende und gegebenenfalls durch C₁-C₄-Alkyl und/oder Hydroxy-substituiertes C₁-C₁₃-Alkanol sind beispielsweise Methanol, Ethanol, Propanol, Isopropanol, Butanol, Isobutanol, sec-Butanol, tert-Butanol, Pentanol, Isopentanol, Neopentanol, tert-Pentanol, Hexanol, 2-Methylpentanol, Heptanol, Octanol, 2-Ethylhexanol, Isooctanol, Nonanol, Isononanol, Decanol, Isodecanol, Undecanol, Dodecanol, Tridecanol, Isotridecanol, 2-Methoxyethanol, 2-Ethoxyethanol, 2-Propoxyethanol, 2-Butoxyethanol, 2-oder 3-Methoxypropanol, 2- oder 3-Ethoxypropanol, 2- oder 3-Propoxypropanol, 2-oder 4-Methoxybutanol, 2- oder 4-Ethoxybutanol, 3,6-Dioxaheptanol, 3,6-Dioxaoctanol, 3,7-Dioxaoctanol, 4,7-Dioxaoctanol, 2- oder 3-Butoxypropanol, 2- oder 4-Butoxybutanol, Ethan-1,2-diol, Propan-1 ,2-diol, Propan-1,3-diol, 3-Oxa-5-hydroxypentanol, 3,6-Dioxa-8-hydroxyoctanol, 3-Oxa-5-hydroxy-2,5-dimethylpentanol oder 3,6-Dioxa-8-hydroxy-2,5,8-trimethyloctanol.

Besonders bevorzugt ist das mindestens eine gegebenenfalls ein oder zwei nicht benachbarte Sauerstoffatome als Etherbindung enthaltende und gegebenenfalls durch C₁-C₄-Alkyl und/oder Hydroxy-substituierte C₁-C₁₃-Alkanol ausgewählt aus Methanol, Ethanol, Propanol, Isopropanol, Butanol, Isobutanol, sec-Butanol, tert-Butanol, Pentanol, Isopentanol, Neopentanol, tert-Pentanol, Hexanol, 2-Methypentanol und Heptanol oder Mischungen daraus.

Ganz besonders bevorzugt sind Butanol, Isobutanol, sec-Butanol und tert-Butanol sowie Mischungen aus Methanol und Butanol.

Ein cyclischer Kohlensäureester ist ein Carbonat der allgemeinen Formel (III) 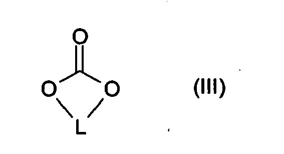 in der
L Ethylen, 1,2- oder 1,3-Propylen oder 1,2-, 1,4-, 2,3- oder 1,3-Butylen bedeutet.
Acyclische Kohlensäureester sind z. B. Diarylcarbonat, Dialkylcarbonat, Arylalkylcarbonat und Dialkenylcarbonat. Bevorzugt ist der acyclische Kohlensäureester ausgewählt aus Carbonaten der allgemeinen Formel (IV)

Z¹O-CO-OZ² (IV)

in der
Z¹ und Z² jeweils unabhängig voneinander Alkyl, Cycloalkyl und Aryl bedeuten. Bevorzugt enthalten die Reste Z¹ und Z² weniger als 13 Kohlenstoffatome. Mehr bevorzugt sind Z¹ und Z² ein C₁-C₈-Alkyl und insbesondere Methyl oder Butyl.

Bevorzugte Dialkylcarbonate sind Dimethylcarbonat, Diethylcarbonat, Dipropylcarbonat, Dibutylcarbonat und Methylbutylcarbonat.

Bevorzugte Arylalkylcarbonate sind Methylphenylcarbonat oder Butylphenylcarbonat.

Geeignete Diarylcarbonate sind z. B. Diphenylcarbonat, Di-(para-tolyl)carbonat, Di-(α-naphtyl)carbonat oder Di-(β-naphtyl)carbonat.

Ein bevorzugtes Dialkenylcarbonat ist Diallylcarbonat.

Besonders bevorzugte Kohlensäureester sind Dimethylcarbonat, Diethylcarbonat, Dibutylcarbonat, Methylbutylcarbonat, Diphenylcarbonat, Propylencarbonat oder Mischungen daraus.

Geeignete Alkali- oder Erdalkalialkanolate sind z. B. Lithium-, Natrium-, Kalium-, Magnesium- oder Calciumsalze der oben näher bezeichneten Alkanole. Die Verwendung von Alkalimethanolaten, insbesondere von Natriummethanolat, ist bevorzugt. Das Alkali- oder Erdalkalialkanolat kann entweder in festem Aggregatzustand oder in gelöster oder suspendierter Form zur Anwendung gelangen.

Bevorzugte Lösungsmittel/Verdünnungsmittel sind in diesem Fall insbesondere die oben näher bezeichneten Alkohole, allein oder als Mischung untereinander. Es können jedoch auch andere an sich bekannte und übliche inerte Verdünnungsmittel zur Anwendung gelangen.

Katalysatoren, die im Reaktionsgemisch enthalten sein können, sind Katalysatoren, die zur Herstellung des Alkoxycarbonylaminotriazins eingesetzt werden. Solche Katalysatoren sind z. B. Phasentransferkatalysatoren, wie sie in Ullmann's Encyclopedia of Industrial Chemistry, 5th Edition, Vol. A19, Seiten 239 bis 248 beschrieben sind. Weitere Katalysatoren können Metallsalze oder -komplexe sein, vorzugsweise Oxide, Chalkogenate, Carbonate oder Halogenide der Alkali-, Erdalkali- oder Übergangsmetalle. Zu nennen sind hier insbesondere Lithiumchlorid, Magnesiumchlorid oder Natriumcarbonat.

Ionische Komponenten, die im Reaktionsgemisch vorliegen, sind im Allgemeinen dissoziierte Salze. Erfindungsgemäß werden diese durch Extraktion mit einem polaren, nicht vollständig mit der im Reaktionsgemisch vorhandenen organischen Phase mischbaren Extraktionsmittel entfernt.

Weiterhin können polare Komponenten erfindungsgemäß aus dem Reaktionsgemisch durch die Extraktion mit dem polaren, nicht vollständig mit der im Reaktionsgemisch vorhandenen organischen Phase mischbaren Extraktionsmittel entfernt werden.

Salze im Reaktionsgemisch können zum Beispiel entstehen, wenn dem alkanolischen Reaktionsgemisch zum Neutralisieren Säure zugegeben wird oder wenn das alkanolische Reaktionsgemisch in eine Säure eingetragen wird. Die Säure kann dabei konzentriert oder mit Wasser verdünnt sein. Eine gleichmäßige Verteilung der Säure im Reaktionsgemisch wird dadurch erreicht, dass während der Dosierung der Säure eine geeignete Durchmischung gewährleistet wird.

Zum Neutralisieren des Reaktionsgemisches können alle üblichen und industriell verfügbaren organischen und anorganischen Säuren in beliebiger Konzentration, vorzugsweise als 30-85 gew.-%ige wässrige Lösungen, verwendet werden. Vorzugsweise verwendet man Mineralsäuren, deren Salze eine hohe Wasserlöslichkeit aufweisen, wie Salpetersäure, Schwefelsäure oder Phosphorsäure. Eine weitere geeignete Säure ist Ameisensäure. Erfindungsgemäß ist die Verwendung von Salpetersäure besonders bevorzugt.

Die Extraktion wird mit einem polaren, nicht vollständig mit der organischen Phase mischbaren Extraktionsmittel durchgeführt, wobei eine alkanolische, Alkoxycarbonylaminotriazin enthaltende Phase und eine polare Phase erhalten wird, die Extraktionsmittel mit darin gelösten ionischen und/oder polaren Komponenten enthält. Nicht vollständig mischbar bedeutet dabei, dass sich zwei Phasen mit unterschiedlicher Zusammensetzung bilden, wobei unter nicht vollständig mischbar auch verstanden wird, dass sich das Extraktionsmittel und die organische Phase überhaupt nicht mischen. Bevorzugt als Extraktionsmittel ist Wasser, besonders bevorzugt ist voll entsalztes Wasser.

Zusätzlich zu dem polaren Extraktionsmittel können bei der Extraktion Phasentrennhilfsmittel zugegeben werden, wie sie zum Beispiel in Ullmann's Encyclopedia of Industrial Chemistry, Sixth Edition, 2000 Electronic Release, Abschnitt Emulsion, Kapitel 6, Breaking of Emulsions beschrieben sind. Phasentrennhilfsmittel sind zum Beispiel organische Lösungsmittel, Stoffe mit Tensidcharakter, wie Phenolsulfonsäurekondensate, Polyimine oder Polyacrylate, Emulsionsspalter und Demulgatoren. Auch Salze können in bestimmten Fällen verwendet werden. Erfindungsgemäß geeignete Phasentrennhilfsmittel sind zum Beispiel Sepabase® der Baker Petrolite, Sugar Land, Texas oder Sokalan® CP, Sokalan® CP5, Sokalan® CP9 und Sokalan® HP25 der BASF AG, Ludwigshafen. Bevorzugt werden jedoch keine Phasentrennhilfsmittel eingesetzt.

Wenn eine Neutralisation des alkanolischen Reaktionsgemisches durchgeführt wird, können diese und die Entfernung der durch die Neutralisation entstehenden Salze durch Extraktion in einem Schritt oder in getrennten Verfahrensschritten erfolgen. Bevorzugt erfolgen die Neutralisation und das Entfernen der durch die Neutralisation entstehenden Salze in zwei Schritten.

Für die Extraktion können die dem Fachmann bekannten Apparate, z. B. Mixer/Settler-Einheiten, Kolonnen mit oder ohne Energieeintrag oder Extraktoren, die auf dem Prinzip der Zentrifugalfeldtrennung basieren, eingesetzt werden. Eine Mixer/Settler-Einheit umfasst im Allgemeinen eine Mischeinheit wie einen Rührbehälter, eine Mischpumpe, eine Düse oder einen statischen/dynamischen Mischer. Weiterhin umfasst die Mixer/Settler-Einheit einen Abscheider, der im Allgemeinen als liegender Behälter mit oder ohne Einbauten ausgeführt ist.

Geeignete Kolonnen, die für die Extraktion eingesetzt werden können, sind z. B. Füllkörper-, Packungs- oder Siebbodenkolonnen. Geeignete Siebbodenkolonnen sind z. B. auch Querstromsiebbodenkolonnen. Als Füllkörper können alle dem Fachmann bekannten Füllkörper verwendet werden. Solche Füllkörper sind zum Beispiel in Klaus Sattler, Thermische Trennverfahren, 2. Auflage, VCH Verlagsgesellschaft mbH, Weinheim, 1995, Seiten 226 bis 229 beschrieben.

Als Packungen eignen sich geordnete oder ungeordnete Packungen. Solche Packungen sind z. B. strukturierte Packungen, Lamellenpackungen, Gewebe, Gestricke oder Gewirke.

Die Füllkörper, Packungen oder Siebböden können aus Metall oder Kunststoff gefertigt sein. Aufgrund der guten Benetzungseigenschaften von Metallen wird bei Wahl der Waschphase als kontinuierliche Phase vorzugsweise Metall als Werkstoff für die Füllkörper, Packungen oder Siebböden verwendet. Besonders geeignete Metalle sind rostfreie Edelstähle.

Neben dem Betrieb der Kolonnen mit Füllkörpern, Packungen oder Böden mit und ohne Pulsation ist auch ein Einsatz ohne Einbauten, z. B. als Sprühkolonne denkbar. Als Beispiele für geeignete, handelsübliche Extraktionskolonnen mit mechanischen Rührsystemen sind Drehscheibenextraktoren, Old-Rushton-Kolonnen, Kühni-Extraktoren, Rührzellenextraktoren, Graesser-Extraktoren zu nennen. Aber auch Zentrifugalextraktoren wie Podbielniak-Extraktoren oder Lurgi-Westfalia-Extraktoren können eingesetzt werden.

Bei der Extraktion kann die das polare, nicht vollständig mit der organischen Phase mischbare Extraktionsmittel enthaltende Waschphase entweder die kontinuierliche oder die disperse Phase der Extraktion bilden. In einer bevorzugten Form der Extraktion bildet die Waschphase die kontinuierliche Phase.

Neben der Verwendung eines einzelnen Extraktionsapparates ist es auch möglich, die Extraktion in mehreren Apparaten durchzuführen. Hierbei kann auch eine Kombination verschiedener Apparatetypen eingesetzt werden. Eine bevorzugte Kombination bilden dabei eine Mixer/Settler-Einheit und eine Füllkörperkolonne. In einer besonders bevorzugten Ausführungsform wird die Extraktion in einer Packungskolonne durchgeführt.

Bei der Extraktion liegt das Phasenverhältnis von polarer zu organischer Phase in einem Bereich von 0,1 bis 2. Bevorzugt ist ein Phasenverhältnis im Bereich von 0,15 bis 1,5, besonders bevorzugt von 0,2 bis 1 und insbesondere von 0,3 bis 0,5.

Bei Verwendung einer Packungskolonne zur Extraktion wird in einer bevorzugten Ausführungsform die polare Phase als Extrakt über den Sumpf der Kolonne abgezogen, das Raffinat - die organische Phase - läuft vorzugsweise als freier Überlauf ab. Der Fremdphasenanteil, d. h. das Alkanol, im Extrakt wird vorzugsweise durch ein Kunststoffgestrick im Sumpf der Kolonne abgetrennt.

Bei Verwendung von Wasser als polarem Extraktionsmittel liegt der Fremdphasenanteil, d. h. der Anteil an nicht gelöstem Wasser, im Raffinat nach der Phasentrennunng im Allgemeinen bei etwa 1 Prozent.

Das Raffinat enthält das gewünschte Produkt. Sollte der Anteil an polaren und ionischen Komponenten im Raffinat größer sein als es die erforderliche Produktspezifikation erlaubt, ist es in einer bevorzugten Ausführungsform möglich, das Raffinat in den Feed zurückzuführen. Dabei kann das Raffinat z.B. entweder direkt in den Feedzulauf zur Extraktion eingespeist werden oder in einen Pufferbehälter, aus dem die Extraktion gespeist wird, zurückgeführt werden.

Die polaren und ionischen Komponenten sind z.B. Alkali- oder Erdalkalisalze, Alkali- oder Erdalkalialkanolate, Säure, cyclische oder acyclische Mono- und/oder Diester der Kohlensäure, Alkan(di)ole sowie polare Melaminderivate. Alkandiole sind z.B. Glykol und Propandiol, polare Melaminderivate sind z.B. Melamin, Mono- und Di-Alkoxycarbonylaminotriazine.

Durch die Möglichkeit, Mono- und Di-Alkoxycarbonylaminotriazine durch die Extraktion aus dem Raffinat zu entfernen, ist eine gezielte Einstellung der Verhältnisse verschiedener Alkoxycarbonylaminotriazine im Raffinat möglich.

Die Extraktion in der Packungskolonne wird im Allgemeinen als Gegenstromextraktion durchgeführt. In einer besonders bevorzugten Ausführungsform wird hierzu das polare Extraktionsmittel oberhalb der Packung und das alkanolische Reaktionsgemisch unterhalb der Packung zugeführt. Innerhalb der Kolonne strömt so das polare Extraktionsmittel durch die Packung in Richtung des Kolonnensumpfes und das alkanolische Reaktionsgemisch durch die Packung in Richtung des Kolonnenkopfes. In der Packung vermischen sich das alkanolische Reaktionsgemisch und das polare Extraktionsmittel, wobei die im alkanolischen Reaktionsgemisch enthaltenen ionischen und/oder polaren Komponenten an das polare Extraktionsmittel abgegeben und so aus dem alkanolischen Reaktionsgemisch entfernt werden.

Die Temperatur, bei welcher die Extraktion durchgeführt wird, liegt vorzugsweise im Bereich von 10 bis 90°C, besonders bevorzugt im Bereich von 15 bis 50°C.

Ein bevorzugter Druck, bei dem die Extraktion durchgeführt wird, ist der Umgebungsdruck. Es ist jedoch auch möglich, die Extraktion bei einem Druck unterhalb des Umgebungsdrucks oder auch bei einem erhöhten Druck durchzuführen. Wenn die Extraktion bei erhöhtem Druck durchgeführt wird, liegt der Druck vorzugsweise im Bereich von 1 bis 10 bar.

Wenn der pH-Wert des Reaktionsgemisches von einem gewünschten pH-Wert abweicht, lässt sich dieser durch Zugabe einer Säure oder einer Base einstellen. Der pH-Wert des Reaktionsgemisches soll im Allgemeinen zwischen 2 und 8, bevorzugt zwischen 2 und 7 und besonders bevorzugt zwischen 4,2 und 6,5 liegen. Die Einstellung des pH-Wertes kann dabei sowohl vor der Extraktion als auch während der Extraktion erfolgen.

Bei einem zu niedrigen pH-Wert lässt sich dieser durch Zugabe einer Base erhöhen. Hierzu sind alle Basen geeignet. Bevorzugt werden NaOH oder Ammoniakwasser (NH₄0H in wässriger Lösung) eingesetzt. Besonders bevorzugt ist Ammoniakwasser.

Ein zu hoher pH-Wert lässt sich durch Zugabe einer Säure auf den gewünschten Wert senken. Hierfür sind sowohl mineralische als auch organische Säuren geeignet. Bevorzugt sind organische Säuren und besonders bevorzugt ist Ameisensäure.

In einer Ausführungsform können im Reaktionsgemisch enthaltene ionische Komponenten zusätzlich durch einen Ionenaustausch an einem Kationentauscher und/oder Anionentauscher entfernt werden. Der Ionenaustausch kann dabei entweder vor der Extraktion oder nach dieser durchgeführt werden.

Der Kationentauscher und/oder der Anionentauscher können zum Beispiel als Festbettionentauscher vorliegen. Es ist auch möglich, dass der Kationentauscher und/oder der Anionentauscher als Granulat vorliegen. Der Kationentauscher und/oder der Anionentauscher können dabei in einem Behälter, z.B. einem Rührkessel, einer Säule, einer Kolonne oder in einem sonstigen, dem Fachmann bekannten Apparat vorliegen. Bevorzugt liegen der Kationentauscher und/oder Anionentauscher in einer Säule vor.

In einer weiteren Ausführungsform ist es auch möglich, den Kationentauscher und/oder Anionentauscher als Granulat dem Reaktor zuzuführen, in welchem das Alkoxycarbonylaminotriazin hergestellt wird. Der Reaktor ist in diesem Fall vorzugsweise ein Rührkessel.

In einer bevorzugten Ausführungsform werden die Alkalimetall- und/oder Erdalkalimetallionen mit einem Kationentauscher aus dem alkanolischen Reaktionsgemisch entfernt.

Weiterhin ist es möglich, mit einem Anionentauscher Anionen zu entfernen. So können zum Beispiel durch die Neutralisation des Reaktionsgemisches mit einer Säure Nitrat-, Sulfat- oder Phosphationen oder auch die Anionen von organischen Säuren, wie Ameisensäure, in dem Reaktionsgemisch enthalten sein, die durch den Ionenaustausch mit dem Anionentauscher entfernt werden.

Eine Regeneration des beladenen Anionentauschers erfolgt vorzugsweise mit verdünnten, mineralischen Laugen. Besonders geeignet zur Regeneration des Anionentauschers ist 5-25%ige Natronlauge.

Eine Regeneration des Kationentauschers erfolgt vorzugsweise mit verdünnten, mineralischen Säuren. Eine geeignete mineralische Säure ist z. B. 5-30%ige Salzsäure.

Zum Durchlaufen mehrerer Zyklen wird sowohl das Anionentauscherharz als auch das Kationentauscherharz im Allgemeinen mit einem Lösungsvermittler zwischen organischer und polarer Phase vorbehandelt. Hierzu wird der Ionentauscher mit einer Substanz gespült, die eine Polarität aufweist, die zwischen der Polarität der organischen und der polaren Phase liegt und vorzugsweise mit beiden Phasen mischbar ist. Zum Beispiel eignet sich Methanol als Lösungsvermittler bei Butanol als organischer Phase und Wasser als polarer Phase. Neben einer Regeneration des Ionentauscherharzes ist auch ein Verwerfen des beladenen Harzes ohne Regeneration denkbar.

Erfindungsgemäß geeignete Anionentauscher sind zum Beispiel stark basische Anionentauscherharze. Bevorzugt sind vernetzte Polystyrol-Harze oder Styrol-Divinylbenzol-Copolymere mit tertiären oder quartären Aminen als funktionelle Gruppe und OH⁻-Ionen als Austauschionen. Unter Austauschionen sind dabei die Ionen zu verstehen, die an die funktionellen Gruppen gebunden sind und gegen die aus der Flüssigkeit zu entfernenden Ionen ausgetauscht werden. Bei kommerziell erhältlichen Anionentauschern liegen die funktionellen Gruppen im Allgemeinen als Salze vor. Hierbei sind zum Beispiel CI⁻-Ionen an die funktionelle Gruppe gebunden. Um den Anionentauscher einsetzen zu können wird dieser in diesem Fall im Allgemeinen zunächst mit NaOH vorbehandelt, um die CI⁻-Ionen gegen OH⁻-Ionen zu tauschen. Geeignete, kommerziell erhältliche Anionentauscher sind zum Beispiel Lewatit® MP62, Lewatit® MP64 oder Lewatit® MP 600 WS der Firma Bayer AG oder auch Amberjet® 4200 CL oder Ambersep® 900 OH der Firma Rohm & Haas Co. Zur Entfernung von Nitrationen sind Ambersep® 900 OH und Lewatit® MP 600 WS bevorzugt, besonders bevorzugt ist Ambersep® 900 OH

Geeignete Kationentauscher sind zum Beispiel stark saure Kationentauscherharze auf Basis einer vernetzten Polystyrol-Matrix oder einer Styrol-Divinylbenzol-Copolymer-Matrix und Sulfonsäure als funktioneller Gruppe mit H⁺-Ionen als Austauschionen. Im Allgemeinen liegen die Kationentauscher ebenso wie die Anionentauscher in ihrer Salzform vor, wenn diese in den Handel gelangen. Um den Kationentauscher einsetzen zu können wird dieser dann im Allgemeinen mit einer Säure, z.B. Schwefelsäure, vorbehandelt, um die Kationen des Salzes durch H⁺-Ionen auszutauschen. Kommerziell erhältliche, geeignete Kationentauscher sind zum Beispiel Lewatit® S2528 oder Lewatit MonoPlus® S100 der Firma Bayer AG, Amberlyst® 40 WET und Amberjet® 1500 H der Firma Rohm & Haas Co. sowie Dowex® N306 von Dow Chemical Co. Zur Entfernung von Natriumionen werden z.B. bevorzugt Amberlyst® 40 WET und Amberjet® 1500 H eingesetzt.

Der Kationentauscher und der Anionentauscher können entweder zusammen als Gemisch, einzeln oder in hintereinander geschalteten Schritten oder Stufen eingesetzt werden. Geeignete Kombinationen geeigneter kommerziell erhältlicher Anionentauscher und Kationentauscher sind bei der Entfernung von Nitratsalzen Ambersep® 900 OH oder Amberjet® 4200 als Anionentauscher und Lewatit® S2528 als Kationentauscher. Bevorzugt ist die Kombination von Ambersep® 900 OH und Lewatit® S2528.

Der Kontakt des.alkanolischen Reaktionsgemisches mit dem Kationentauscher und/oder Anionentauscher kann z. B. dadurch erfolgen, dass der Kationentauscher und/oder Anionentauscher z. B. in den Reaktor oder in einen Rührbehälter zum Reaktionsgemisch zugegeben werden, oder dadurch, dass das Reaktionsgemisch einen kontinuierlichen Ionentauscher, wobei der Ionentauscher z. B. als Packung in einem Festbett vorliegt, durchströmt.

Die Zugabe des Ionentauscherharzes in den Reaktionsbehälter ist insbesondere dann möglich, wenn das mindestens eine Alkoxycarbonylaminotriazin diskontinuierlich hergestellt wird. In diesem Fall erfolgen bevorzugt sowohl die Herstellung des mindestens einen Alkoxycarbonylairinotriazins als auch gegebenenfalls eine Neutralisation des Reaktionsgemisches und die Entfernung der ionischen Komponenten durch Ionentausch im gleichen Behälter.

In einer Ausführungsform kann das Verfahren als zusätzlichen Schritt das Aufkonzentrieren der organischen, mindestens ein Alkoxycarbonylaminotriazin enthaltenden Phase umfassen.

Das Aufkonzentrieren kann dabei durch thermische oder mechanische Verfahren erfolgen. Geeignete thermische Verfahren zum Aufkonzentrieren sind zum Beispiel Verdampfung, Destillation, Rektifikation, Trocknung, vorzugsweise Sprühtrocknung, oder Kristallisation. Geeignete mechanische Verfahren sind insbesondere Membrantrennverfahren, zum Beispiel Pervaporation oder Permeation sowie Filtration, wenn das mindestens eine Alkoxycarbonylaminotriazin als Suspension vorliegt. Die Verfahren zum Aufkonzentrieren können jeweils einzeln oder in Kombination zur Anwendung kommen. Es kann auch jedes weitere geeignete dem Fachmann bekannte Verfahren zur Aufkonzentrierung eingesetzt werden. Bevorzugte Verfahren zum Aufkonzentrieren sind Destillation und Sprühtrocknung.

Bei der Aufkonzentrierung durch Destillation kann diese vor oder nach der Extraktion durchgeführt werden. Wenn zusätzlich ein Ionentausch durchgeführt wird, kann die Destillation auch vor oder nach dem Ionenaustausch erfolgen, wobei die Durchführung der Destillation vor dem Ionentausch bevorzugt ist, da durch die Destillation der das Alkoxycarbonylaminotriazin enthaltende Volumenstrom verringert wird und so kleinere Apparate zur Durchführung des Ionentausches eingesetzt werden können. Hierdurch werden sowohl die Investitions- als auch die Betriebskosten für den Ionenaustausch gesenkt..

Das Aufkonzentrieren der organischen, Alkoxycarbonylaminotriazin enthaltenden Phase durch Destillation kann kontinuierlich oder diskontinuierlich erfolgen.

Zur kontinuierlichen Destillation können herkömmliche, dem Fachmann bekannte kontinuierliche Verdampfer eingesetzt werden. Geeignete Verdampfer zur kontinuierlichen Destillation sind z. B. Umlaufverdampfer, wie Robert-Selbstumlaufverdampfer, Schnellumlaufverdampfer mit schrägen Verdampferrohren, Zwangsumlaufverdampfer mit au-βen liegendem Verdampferbündei, Umlaufverdampfer mit in Kammern unterteiltem Siederaum oder Zwangsumlaufverdampfer mit liegendem Heizkörper. Weitere geeignete kontinuierliche Verdampfer sind z. B. Fallfilmverdampfer, Dünnschichtverdampfer oder Kestnerverdampfer.

Weiterhin kann das Aufkonzentrieren der organischen Phase durch Destillation in einer Kolonne erfolgen. Die Erwärmung auf Verdampfungstemperatur kann dabei am Kolonnensumpf erfolgen oder in einem außerhalb der Kolonne liegenden Wärmetauscher. Geeignete Kolonnen sind z. B. Packungskolonnen, Füllkörperkolonnen oder Bodenkolonnen. Geeignete Packungen, Füllkörper oder Böden sind dabei alle dem Fachmann bekannten Packungen, Füllkörper oder Böden.

Eine diskontinuierliche Aufkonzentrierung durch Destillation kann z. B. in einem Rührbehälter erfolgen. Dabei kann die Destillation auch in dem Behälter durchgeführt werden, in dem die Reaktion zu Alkoxycarbonylaminotriazin durchgeführt wird. Bevorzugt erfolgt die Aufkonzentrierung durch Destillation in einem zusätzlichen Rührbehälter.

Sowohl beim kontinuierlichen als auch beim diskontinuierlichen Verfahren fallen der Alkoxycarbonylaminotriazin enthaltende Produktstrom als flüssige Phase und ein mindestens ein Alkanol, Carbonat und Wasser enthaltender Brüdenstrom an. Bei Verwendung eines von Wasser unterschiedlichen polaren Extraktionsmittels bei der Entfernung von durch die Neutralisation entstehenden Salzen durch Extraktion ist im Brüden entweder anstelle des Wassers oder zusätzlich das polare Extraktionsmittel enthalten.

Die Aufkonzentrierung der organischen, Alkoxycarbonylaminotriazin enthaltenden Phase durch Destillation führt in einer besonders bevorzugten Ausführungsform zu einem Produktstrom, welcher 45-60 Gew.-% Alkoxycarbonylaminotriazin enthält.

Abhängig vom gewünschten Produktstrom ist es jedoch auch möglich, durch die Destillation einen Produktstrom zu erhalten, der einen kleineren oder auch einen größeren Anteil an Alkoxycarbonylaminotriazin enthält.

In einer weiteren Verfahrensvariante werden bei der Aufkonzentrierung der organischen, mindestens ein Alkoxycarbonylaminotriazin enthaltenden Phase durch Destillation die organische Phase und die polare Phase abgetrennt. Hierzu wird die organische, mindestens ein Alkoxycarbonylaminotriazin enthaltende Phase einer Destillationskolonne zugeführt. Die Destillationskolonne umfasst dabei vorzugsweise einen Verstärkungsteil und einen Abtriebsteil. Der Zulauf der organischen, mindestens ein Alkoxycarbonylaminotriazin enthaltenden Phase erfolgt vorzugsweise über einen Seitenzulauf im Verstärkungsteil.

Am Sumpf der Destillationskolonne wird die aufkonzentrierte, organische, Alkoxycarbonylaminotriazin enthaltende Phase gewonnen.

In einer bevorzugten Ausführungsform wird das so gewonnene Sumpfprodukt in einem Wärmetauscher zum Erwärmen der entsalzten organischen Phase, die der Destillationskolonne als Feed zugeführt wird, genutzt.

Über den Kopf der Destillationskolonne werden Alkanole, gegebenenfalls Leichtsieder und Wasser und/oder polares Extraktionsmittel abgezogen. Dieser Strom wird in einer besonders bevorzugten Ausführungsform anschließend einem Phasenscheider zugeführt, in welchem die polare Phase von der organischen Phase getrennt wird. Die organische Phase wird vorzugsweise als Rücklauf erneut der Destillationskolonne an deren Kopf zugeführt.

In einer besonders bevorzugten Ausführungsform umfasst die Destillationskolonne einen vorzugsweise im Abtriebsteil angeordneten Seitenabzug, über welchen ein vorzugsweise dampfförmiger und im Wesentlichen wasserfreier, Carbonat und Alkanol enthaltender Strom abgezogen wird. Eine besonders bevorzugte Position des Seitenabzuges ist direkt oberhalb des Kolonnensumpfes bzw. direkt unterhalb der trennwirksamen Einbauten in der Kolonne.

Vorteile dieser Betriebsweise liegen in der Rückgewinnung eines Großteils der Carbonate über Seitenabzug zum Wiedereinsatz in der Reaktion, der Reduzierung des Carbonatgehaltes im Produktstrom und in der Rückgewinnung von wasserfreien schwersiedenden Alkanolen, z.B. n-Butanol, über den Seitenabzug.

In einer bevorzugten Ausführungsform umfasst die Destillationskolonne 8 bis 22 theoretische Trennstufen.

Das Rücklaufverhältnis der organischen Phase am Kopf der Kolonne liegt vorzugsweise im Bereich zwischen 0,2 und 3 kg/kg.

Die Destillationskolonne wird vorzugsweise mit einem Druck im Bereich zwischen 20 und 2000 mbar am Kopf der Kolonne betrieben. Der bevorzugte Bereich, in welchem die Kolonne betrieben wird, liegt zwischen 50 und 950 mbar.

In einer weiteren Verfahrensvariante ist es möglich, zusätzlich zum Aufkonzentrieren der Alkoxycarbonylaminotriazin enthaltenden Phase durch Destillation oder anstelle des Aufkonzentrierens durch Destillation als weiteren Verfahrensschritt eine Sprühtrocknung vorzusehen. Durch die Sprühtrocknung wird pulverförmiges Alkoxycarbonylaminotriazin erzeugt.

Die Sprühtrocknung erfolgt dabei vorzugsweise in einem Sprühtrockner wie er dem Fachmann bekannt ist. So können für die Sprühtrocknung beispielsweise handelsübliche Sprühtrockner mit Zerstäuberscheibe, Einstoffdüse oder Zweistoffdüse eingesetzt werden. Der Betrieb kann je nach Bauart im Gleichstrom oder im Gegenstrom erfolgen. Bevorzugt wird eine Zweistoffdüse eingesetzt, bei der die flüssige, mindestens ein Alkoxycarbonylaminotriazin enthaltende Phase drucklos mit Hilfe eines Stickstoffstromes zerstäubt wird. Der Stickstoffstrom weist dabei einen Druck im Bereich von 1 bis 10 bar, bevorzugt im Bereich von 2 bis 5 bar und besonders bevorzugt im Bereich von 2,5 bis 5 bar auf. Der Stickstoff wird dabei vorzugsweise als Kreisgas eingesetzt.

Die Sprühtrocknung wird vorzugsweise bei einer Temperatur im Bereich von 50 bis 250°C, bevorzugt bei einer Temperatur von 55 bis 150°C und besonders bevorzugt bei einer Temperatur im Bereich von 60 bis 100°C und bei Umgebungsdruck oder einem Über- oder Unterdruck von bis zu +/- 0,01 MPa, bezogen auf den Umgebungsdruck, durchgeführt.

Das bei der Sprühtrocknung erzeugte, pulverförmige Produkt kann beispielsweise in einem Gewebefilter üblicher Bauart, wie Kerzenfilter, Sackfilter, Schlauchfilter oder anderen dem Fachmann bekannten Filtern oder in einem Zyklon abgetrennt werden. Als Filtermaterial für einen Gewebefilter eignet sich zum Beispiel Polytetraflourethylen, Silikon oder Polyester. Bevorzugt ist Polyester.

Die Reinigung des als Kreisgas eingesetzten Stickstoffes erfolgt vorzugsweise in einem Wäscher. Dabei ist jeder beliebige, dem Fachmann bekannte Wäscher einsetzbar.

### Beispiele

### Beispiel 1

Ein unter Verwendung von Dimethylcarbonat hergestelltes, Alkoxycarbonylaminotriazin enthaltendes Reaktionsgemisch wird nach der mit 30%iger HNO₃ durchgeführten Neutralisation aufgearbeitet. Hierzu werden 63,6 kg/h der in einem Phasenscheider abgetrennten organischen homogenen Phase mit einem Wassergehalt von 13%, einem Na-Gehalt 3120 mg/kg und einem MO₃⁻-Gehalt von 7440 mg/kg in einer Packungskolonne mit 22,3 kg/h voll entsalztem Wasser im Gegenstrom gewaschen. Die Belastung der Kolonne liegt dabei bei 19,2 m³/m²h, was 75% des Flutpunktwertes entspricht. Die Temperatur, bei welcher gewaschen wird, beträgt 21°C. Es ergeben sich ein Raffinatstrom von 60,7 kg/h und ein Extraktstrom von 28,2 kg/h. Dabei stellt der Raffinatstrom das Alkoxycarbonylaminotriazin enthaltende Wertprodukt dar. Die Analyse der Natriumionen und Nitrationen der gewaschenen organischen Phase ergibt eine Konzentration von 1 mg/kg Na⁺ und 48 mg/kg No₃⁻. Der Wassergehalt der organischen Phase beträgt 19,7%.

### Beispiel 2

Es werden 31,9 kg/h der organischen, Alkoxycarbonylaminotriazin enthaltenden Phase nach Neutralisation mit 30%iger HNO₃ der Packungskolonne zugeführt. Die organische Phase wird mit 12,5 kg/h voll entsalztem Wasser im Gegenstrom in der Kolonne gewaschen. Die Belastung der Kolonne liegt dabei bei 9,9 m³/m²h, was 37% des Flutpunktwertes entspricht. Aus der Packungskolonne werden ein Alkoxycarbonylaminotriazin enthaltender Raffinatstrom von 28,2 kg/h und ein Extraktstrom von 13,9 kg/h abgezogen. Der Wassergehalt der gewaschenen organischen Phase beträgt 19,0%. Die Konzentration an Natriumionen beträgt 1 mg/kg, die Konzentration an Nitrationen 36 mg/kg.

### Beispiel 3

Ein unter Verwendung von Ethylencarbonat hergestelltes Reaktionsgemisch wird nach Neutralisation mit 30%iger HNO₃ aufgearbeitet. Hierzu werden 17,10 kg/h der abgetrennten organischen, homogenen, Alkoxycarbonylaminotriazin enthaltenden Phase mit einem Wassergehalt von 10%, einem Na-Gehalt von 2640 mg/kg und einem NO₃⁻-Gehalt von 7320 mg/kg in einer Packungskolonne mit 6,0 kg/h Wasser im Gegenstrom gewaschen. Die Belastung der Kolonne liegt dabei bei 20,6 m³/m²h, was 59% des Flutpunktwertes entspricht. Die Temperatur, bei welcher die Waschung durchgeführt wird, beträgt 21 °C. Es ergibt sich ein Alkoxycarbonylaminotriazin enthaltender Raffinatstrom von 16,7 kg/h und ein Extraktstrom von 6,5 kg/h. Der Raffinatstrom enthält 2 mg/kg Natriumionen und 12 mg/kg Nitrationen. Der Wassergehalt des Raffinatstroms beträgt 16,5%.

## Patentansprüche

1. Verfahren zur Aufbereitung eines alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisches, bei welchem polare und/oder ionische Komponenten durch Extraktion mit einem polaren, nicht vollständig mit der im Reaktionsgemisch vorhandenen organischen Phase mischbaren Extraktionsmittel entfernt werden, wodurch eine alkanolische, mindestens ein Alkoxycarbonylaminotriazin enthaltende Phase und eine polare, Extraktionsmittel mit darin gelösten polaren und/oder ionischen Komponenten enthaltende Phase erhalten werden, **dadurch gekennzeichnet, dass** die Extraktion in einer Mixer/Settler-Einheit, einer Extraktionskolonne, einem auf Basis der Zentrifugalfeldtrennung basierenden Extraktor oder Kombinationen daraus durchgeführt wird.

2. Verfahren nach Anspruch 1, **dadurch gekennzeichnet, dass** die Kolonne eine Füllkörper-, Packungs- oder Bodenkolonne ist.

3. Verfahren nach Anspruch 1 oder 2, **dadurch gekennzeichnet, dass** eine gerührte oder gepulste Kolonne eingesetzt wird.

4. Verfahren nach einem der Ansprüche 1 bis 3, **dadurch gekennzeichnet, dass** die Extraktion bei einer Temperatur im Bereich von 20 bis 80°C durchgeführt wird.

5. Verfahren nach einem der Ansprüche 1 bis 4, **dadurch gekennzeichnet, dass** das Phasenverhältnis von polarer zu organischer Phase im Bereich von 0,1 bis 2 liegt.

6. Verfahren nach einem der Ansprüche 1 bis 5, **dadurch gekennzeichnet, dass** das polare Extraktionsmittel Wasser ist.

7. Verfahren nach einem der Ansprüche 1 bis 6, **dadurch gekennzeichnet, dass** das Verfahren als weiteren Schritt das Aufkonzentrieren der organischen, mindestens ein Alkoxycarbonylaminotriazin enthaltenden Phase umfasst.

8. Verfahren nach einem der Ansprüche 1 bis 7, **dadurch gekennzeichnet, dass** das Alkali- oder Erdalkalialkanolat Natriummethanolat ist.

9. Verfahren nach einem der Ansprüche 1 bis 8, **dadurch gekennzeichnet, dass** das C₁-C₁₃-Alkanol Butanol oder eine Mischung aus Methanol und Butanol ist.

10. Verfahren nach einem der Ansprüche 1 bis 9, **dadurch gekennzeichnet, dass** das alkanolischen Reaktionsgemisch vor der Extraktion durch Zugabe von Säure oder durch Eintragen in eine Säure neutralisiert wird.

11. Verfahren nach Anspruch 10, **dadurch gekennzeichnet, dass** die zur Neutralisation zugegebene Säure Salpetersäure ist.

12. Verfahren nach einem der Ansprüche 1 bis 11, **dadurch gekennzeichnet, dass** vor oder während der Extraktion der pH-Wert des Reaktionsgemisches durch Zugabe von Säure oder Base eingestellt wird.

13. Verfahren nach Anspruch 12, **dadurch gekennzeichnet, dass** die Base Ammoniakwasser ist.

14. Verfahren nach Anspruch 12, **dadurch gekennzeichnet, dass** die Säure eine organische Säure, vorzugsweise Ameisensäure, ist.

15. Verfahren nach einem der Ansprüche 1 bis 14, **dadurch gekennzeichnet, dass** das Verfahren als abschließenden Schritt die Herstellung eines im Wesentlichen Alkoxycarbonylaminotriazin enthaltenden Pulvers durch Sprühtrocknung umfasst.

16. Verfahren nach Anspruch 15, **dadurch gekennzeichnet, dass** die Sprühtrocknung bei einer Temperatur im Bereich von 50 bis 250°C durchgeführt wird.

## Claims

1. A process for working up an alkanolic reaction mixture which is obtained in the preparation of alkoxycarbonylaminotriazines and comprises at least one alkoxycarbonylaminotriazine, at least one cyclic and/or acyclic carbonic ester, at least one C₁-C₁₃-alkanol which optionally comprises one or two oxygen atoms as an ether bond and is optionally substituted by C₁-C₄-alkyl and/or hydroxyl, and also at least one alkali metal or alkaline earth metal alkoxide, with or without melamine and with or without catalyst, in which polar and/or ionic components are removed by extraction with a polar extractant which is not entirely miscible with the organic phase present in the reaction mixture, to obtain an alkanolic phase comprising at least one alkoxycarbonylaminotriazine and a polar phase comprising extractant with polar and/or ionic components dissolved therein, wherein the extraction is carried out in a mixer/settler unit, an extraction column, an extractor based on centrifugal field separation, or combinations thereof.

2. The process according to claim 1, wherein the column has random packing, structured packing or trays.

3. The process according to claim 1 or 2, wherein a stirred or pulsed column is used.

4. The process according to any of claims 1 to 3, wherein the extraction is carried out at a temperature in the range from 20 to 80°C.

5. The process according to any of claims 1 to 4, wherein the phase ratio of polar to organic phase is in the range from 0.1 to 2.

6. The process according to any of claims 1 to 5, wherein the polar extractant is water.

7. The process according to any of claims 1 to 6, which comprises, as a further step, the concentration of the organic phase comprising at least one alkoxycarbonylaminotriazine.

8. The process according to any of claims 1 to 7, wherein the alkali metal alkoxide or alkaline earth metal alkoxide is sodium methoxide.

9. The process according to any of claims 1 to 8, wherein the C₁-C₁₃-alkanol is butanol or a mixture of methanol and butanol.

10. The process according to any of claims 1 to 9, wherein the alkanolic reaction mixture is neutralized before the extraction by addition of acid or by introduction into an acid.

11. The process according to claim 10, wherein the acid added for neutralization is nitric acid.

12. The process according to any of claims 1 to 11, wherein, before or during the extraction, the pH of the reaction mixture is adjusted by adding acid or base.

13. The process according to claim 12, wherein the base is aqueous ammonia.

14. The process according to claim 12, wherein the acid is an organic acid, preferably formic acid.

15. The process according to any of claims 1 to 14, which comprises, as a final step, the preparation of a powder comprising substantially alkoxycarbonylaminotriazine by spray-drying.

16. The process according to claim 15, wherein the spray-drying is carried out at a temperature in the range from 50 to 250°C.

## Revendications

1. Procédé de traitement d'un mélange réactionnel alcanolique, formé lors de la fabrication d'alcoxycarbonylaminotriazines, contenant au moins une alcoxycarbonylaminotriazine, au moins un ester d'acide carbonique cyclique et/ou acyclique, au moins un alcanol en C₁-C₁₃ contenant éventuellement un ou deux atomes d'oxygène sous la forme d'une liaison éther et éventuellement substitué par un alkyle en C₁-C₄ et/ou un hydroxy, ainsi qu'au moins un alcanolate alcalin ou alcalino-terreux, éventuellement de la mélamine et éventuellement un catalyseur, selon lequel des composants polaires et/ou ioniques sont éliminés par extraction avec un agent d'extraction polaire, non complètement miscible avec la phase organique présente dans le mélange réactionnel, une phase alcanolique contenant au moins une alcoxycarbonylaminotriazine et une phase polaire contenant l'agent d'extraction dans lequel les composants polaires et/ou ioniques sont dissous étant ainsi obtenues, **caractérisé en ce que** l'extraction est réalisée dans une unité mélangeur-décanteur, dans une colonne d'extraction, dans un extracteur fondé sur une séparation en champ centrifuge ou dans une de leurs combinaisons.

2. Procédé selon la revendication 1, **caractérisé en ce que** la colonne est une colonne à corps de remplissage, à garnissage ou à plateaux.

3. Procédé selon la revendication 1 ou 2, **caractérisé en ce qu'**une colonne agitée ou pulsée est utilisée.

4. Procédé selon l'une quelconque des revendications 1 à 3, **caractérisé en ce que** l'extraction est réalisée à une température dans la plage allant de 20 à 80 °C.

5. Procédé selon l'une quelconque des revendications 1 à 4, **caractérisé en ce que** le rapport de phase entre la phase polaire et la phase organique se situe dans la plage allant de 0,1 à 2.

6. Procédé selon l'une quelconque des revendications 1 à 5, **caractérisé en ce que** l'agent d'extraction polaire est l'eau.

7. Procédé selon l'une quelconque des revendications 1 à 6, **caractérisé en ce que** le procédé comprend en tant qu'étape supplémentaire la concentration de la phase organique contenant au moins une alcoxycarbonylaminotriazine.

8. Procédé selon l'une quelconque des revendications 1 à 7, **caractérisé en ce que** l'alcanolate alcalin ou alcalino-terreux est le méthanolate de sodium.

9. Procédé selon l'une quelconque des revendications 1 à 8, **caractérisé en ce que** l'alcanol en C₁-C₁₃ est le butanol ou un mélange de méthanol et de butanol.

10. Procédé selon l'une quelconque des revendications 1 à 9, **caractérisé en ce que** le mélange réactionnel alcanolique est neutralisé avant l'extraction par ajout d'un acide ou par introduction dans un acide.

11. Procédé selon la revendication 10, **caractérisé en ce que** l'acide ajouté pour la neutralisation est l'acide nitrique.

12. Procédé selon l'une quelconque des revendications 1 à 11, **caractérisé en ce que** le pH du mélange réactionnel est ajusté avant ou pendant l'extraction par ajout d'un acide ou d'une base.

13. Procédé selon la revendication 12, **caractérisé en ce que** la base est l'ammoniaque.

14. Procédé selon la revendication 12, **caractérisé en ce que** l'acide est un acide organique, de préférence l'acide formique.

15. Procédé selon l'une quelconque des revendications 1 à 14, **caractérisé en ce que** le procédé comprend en tant qu'étape finale la fabrication d'une poudre contenant principalement une alcoxycarbonylaminotriazine par séchage par pulvérisation.

16. Procédé selon la revendication 15, **caractérisé en ce que** le séchage par pulvérisation est réalisé à une température dans la plage allant de 50 à 250 °C.
